# EUROPEAN PATENT SPECIFICATION

(11) **EP 3 013 282 B1**
(45) Date of publication and mention of the grant of the patent: **02.08.2017**
(21) Application number: 13737164.7
(22) Date of filing: 27.06.2013
(51) Int. Cl.: A61F 2/30, A61F 2/60, A61F 2/50, A61F 2/66, A61F 2/76, A61F 2/80

(54) **A LEG PROSTHESIS AND A METHOD FOR MANUFACTURING THE SAME**
BEINPROTHESE UND VERFAHREN ZU DEREN HERSTELLUNG
PROTHESE DE JAMBE ET SON PROCÉDÉ DE FABRICATION

(43) Date of publication of application: 04.05.2016
(73) Proprietor: SwissLeg Sagl, 6900 Lugano (CH)
(72) Inventor: ISMAIL, Mohammad Shlash Abdallah, 6900 Lugano (CH)
(74) Representative: Zardi, Marco
(86) International application number: PCT/EP2013/063490
(87) International publication number: WO 2014/206470

(56) References cited:
- DE-U1- 8 415 257
- US-A- 4 459 709
- US-A- 5 336 270
- US-A- 5 534 034

## Description

### Field of the invention

The invention relates to a leg prosthesis. More in particular, the invention relates to a leg prosthesis which combines an inexpensive structure with a good performance in restoring the mobility of the patient.

### Prior art

Numerous embodiments of leg prosthesis are known in the art. The most advanced solutions use a true bionic technology, for example including advanced materials like alloys or carbon graphite, computer-controlled motors, sensor means adapted to sense the conditions of use, and control systems based on artificial intelligence.

A motorized prosthetic device is described for example in WO 2012 047721, disclosing a prosthetic or orthotic device comprising: a first segment including a joint member and a second segment coupled with the first segment via the joint member; an actuator configured to control motion of the second segment relative to the first segment; a control system comprising a reactive layer which commands and controls motion of the second segment relative to the first segment; and an inference layer. The inference layer is configured to identify a locomotion activity of a user based on a set of data and transmit information regarding the locomotion activity to the reactive layer to control motion of the second segment relative to the first segment.

These systems aim to recreate functionality of the limb in the best possible way and using the best available technologies. However, they are by far too expensive for a wide distribution and only a limited number of persons can benefit from them.

The prior art also comprises less sophisticated leg prosthesis, which aim to provide a fair good performance and a life like feeling at a more affordable cost.

For example, US 5,800,565 discloses a leg prosthesis comprising: a thigh member; a lower leg member; a knee part having an upper knee member, a lower knee member and a joint connecting said knee members. The leg prosthesis also includes a swing phase control device having a first end connected to the upper knee member and a second end connected to the lower knee member, and a connection unit allowing a relative movement of the connected members in a direction inclined with respect to the leg axis. This allows a relative adjustment of the position of the members, whereby a selection of the correct prosthesis for a thigh amputated person is made possible by testing different members.

US 7,867,286 discloses a prosthesis system including a ventilated shell, and a substantially compliant, ventilated spacer element that defines a first surface having a frictional feature. The spacer element is arranged to secure to an internal surface of the shell. The prosthesis system also includes a connector that is secured to a distal end of the shell, and a retainer supported by the connector so as to extend into a cavity formed by the shell.

US 6,197,067 discloses an artificial limb having an endoskeletal component which is of generally constant H-shaped cross section and is formed by injection molding from thermoplastics material; the limb has a foot keel which is integrally molded with the shin component. The constant cross section of the shin component allows it to be cut to a required length and clamped to an upper limb component.

DE 84 15 257 U1, which is regarded as the closest prior art, discloses a leg prosthesis comprising a hollow shank of expanded stiff plastic material for receiving the lower leg stump, a prosthetic foot and a plastic intermediate piece connecting the shank and the foot.
The foot members can be realized according to various embodiments. For example the foot member may include a multiaxial joint to imitate the flexibility of the ankle.
All the above systems, however, have been designed and developed mainly for the needs of the western countries of the world, and they are still too expensive for millions of people living the less developed countries. In addition to the cost of the prosthetic limb, also the fitting requires resources which are generally not available in said countries.
To date, the only solution which is available to people living in the less developed or emerging countries is represented by rigid prosthesis comprising a thigh member, a leg member and a foot member rigidly screwed on to another. The thigh member and the foot member may be available in some different sizes, normally limited to adult or child; the leg member may also be available in a limited number of lengths, so that the prosthesis can be manufactured locally, by assembling components imported from overseas.
This kind of prosthesis is used by humanitarian organizations and provides a first relief to victims of a limb loss, but the performance and degree of customization are poor. In fact, the artificial leg is totally rigid; a limited flexibility is normally provided by adoption of a flexible foot member. Another disadvantage of the available low cost leg prosthesis is that the weight is almost totally transferred to the knee of the patient, causing a relevant stress.
It should be noted that the need for artificial legs come mostly from the less developed countries and the emerging countries of Africa, Latin America and Asia. Millions of amputees exist in these countries as a consequence of diseases or traumatic events which are unfortunately more common than in other parts of the world; the loss of a limb can make it extremely difficult for victims to provide for themselves and their families. The prior art does not provide a satisfactory solution to this problem. Transferring to the developing countries the advanced prosthetics engineered for the western world is difficult, due to lack of materials and resources especially in the more isolated areas; the current low-cost prosthetics are not able to restore the patient's mobility in a significant way. Furthermore, people living in isolated areas of less developed countries can hardly reach a hospital or other specialized facilities which, can only be found in the main cities.

Another disadvantage of the prior art low cost prosthesis is that they follow a pre-cast technique, which means that the prosthesis is cast according to a standard shape and dimensions, and the prosthetic alignment is performed afterwards. Said step of prosthetic alignment however requires facilities which may be unavailable in the developing countries.

### Summary of the invention

The aim of the invention is to overcome the above drawbacks and limits of the prior art, and provide an inexpensive but satisfactory leg prosthesis. Another aim of the invention is to provide a leg prosthesis which is particularly adapted to meet the high demand for prosthetics which come from the developing countries, taking into account the limited resources available in said countries. A further aim of the invention is to provide a leg prosthesis that can be manufactured on site using limited resources, namely the resources that are normally available in developing countries.

The above aims are reached with a leg prosthesis according to the attached claims. The leg prosthesis comprises a lower leg member and a foot member, wherein said lower leg member is moulded in a single piece; said leg member is a single-piece hollow body comprising an upper portion suitable to accommodate an amputation stump of a patient; a middle portion which forms a hollow pylon of the leg member; a lower portion for connection with the foot member; said leg prosthesis also comprises an adaptor which is received in a seat of the lower leg member, and said foot member is fixed to said adaptor, characterized in that lower leg member is made of a flexible material and said middle portion has the smallest outside dimension but the greatest wall thickness compared to said upper and lower portions of the same leg member.
In a preferred embodiment, the adaptor is fixed to the lower leg member via a male/female tight connection. For example, said adaptor comprises a male part which is tightly fitted into said seat of the lower leg member. Preferably, said adaptor is received in the seat of the lower leg member with a conical fitting. Preferably, the cone angle of said fitting is between 20° and 50°, more preferably between 30° and 40°.
In a preferred embodiment, the adaptor comprises a tapered head, for example having a frusto-conical shape. Accordingly, the leg member comprises a conical seat dimensioned to match the head of the adaptor with a suitable tight fitting.
The lower leg member is advantageously flexible in the direction of the forward movement of a patient using the prosthesis. Preferred materials for the leg member are polypropylene (PP) or a copolymer of polypropylene and polyethylene (PE). Preferably, said copolymer contains 10% to 50% polyethylene, more preferably around 20%.
The physical properties of PP make it ideal for use in prosthetics, having good flexibility and a high melting point, making it resistant to warping. Because of its extensive use, PP can be found throughout the developing world, where it takes the form of PP rods, or can be imported at a low cost. The adaptor, on the other hand, is preferably made of inelastic material.

The aforesaid leg member is substantially a single-piece hollow body. Said leg member comprises an upper portion forming a shell for receiving the amputation stump, a middle portion, and a lower portion for connection with the foot member.

Said upper portion of the leg member is designed to accommodate the amputation stump of the patient. This upper portion may be fabricated by using a cast of the patient, according to a process that will be further explained hereinbelow.

Said middle portion of the leg member forms a hollow pylon, with a suitable thickness to support the weight of the patient. This hollow pylon is substantially the load-bearing part of the leg member. Said middle portion has the smallest outside dimension (e.g. diameter if cylindrical) but the greatest thickness than other parts of the leg member. Hence, it is the slimmest part of the leg prosthesis in appearance, although it is also the most robust section.

The upper portion has preferably a cone-shaped wall portion at the transition with the hollow pylon. Said cone-shaped wall joins the larger diameter of the upper portion with the smaller diameter of the hollow pylon, and serves substantially to uniformly distribute the load.

The lower portion of the leg member is generally larger but thinner than the hollow pylon, and provides room for the internal adaptor which, in turn, supports the foot member. Preferably, the adaptor is entirely received in the lower portion of the leg member, so that no part of the adaptor remains visible when the leg prosthesis is assembled.

The foot member is fixed to the adaptor by means of fixation means. Said fixation means preferably comprise a fixating screw.

It should be noted that, preferably, the leg prosthesis of the invention consist of the above described leg member, foot member, adaptor and fixation means between the adaptor and the foot member, without additional structural components, apart from cosmetic coating. Hence it consists of few elements to the advantage of cost and easy manufacturing.

A preferred process for the manufacturing of the leg prosthesis comprises at least the following steps:
- taking an negative cast of an amputation stump of a patient;
- making a positive cast of the upper part of said leg member starting from said negative cast;
- coating said positive cast with a soft liner, thus obtaining a mould;
- fitting said mould with an adaptor, said adaptor being configured to provide a mould for the realization of middle and lower portions of said leg member;
- making the leg member by draping previously heated sheets of a plastic material onto the mould;
- removing said adaptor and removing the material of the positive cast, thus obtaining a single-piece hollow leg member.

Preferably the step of draping heated sheets of plastic onto the mold is performed under vacuum. Said soft liner, in a preferred embodiment, is any of: EVA, Evalux, TP foam, light PE.

After the hollow leg member is completed, it can be finished according to the needs and then the foot member is mounted with the help of the adaptor.

The inventive leg prosthesis provides a satisfactory degree of customization while, on the other hand, it comprises only a limited number of parts, namely the leg member, the foot member, the adaptor and the fixating screw.

The flexibility is given by the leg member, which can be made with inexpensive process and equipment, easily available also in less developed countries or in emerging countries.

The foot member and the adaptor can be standardized to reduce costs. In particular, the foot member can be a basic prosthetic foot such as the SACH (solid ankle cushioned heel) type, which is one of the lowest cost prosthetic feet on the market. Thanks to the invention, an acceptable restoration of the patient's mobility is obtained, despite the use of such a simple and inexpensive foot member.

Another advantage is a good distribution of load, without an excessive stress for the knee. Hence the leg prosthesis is well tolerated by the patient.

A great advantage is that the leg prosthesis of the invention can be produced by a relatively simple and sequential manufacturing process, which does not require expensive resources and can be completed in a few hours from measurement of the stump to fitting of the finished prosthesis. The process requires electricity and a heat source (for example an oven) adapted to reach the temperature for thermo moulding, for example around 300 °C. A suitably trained practitioner can take care of the whole process, without the need of expensive facilities. Polypropylene, for example, is cheap and easily mouldable on any shape required. Hence, the leg prosthesis of the invention can be produced on site also in remote locations and in developing countries where the need for limb prosthesis is higher. The necessary machines can be installed in a mobile laboratory to reach a higher number of amputees.

These and other features and advantages of the invention shall become more evident from the following detailed description and with the help of the attached figures.

### Description of the figures

Fig. 1 is a cross sectional view of a leg prosthesis according to an embodiment of the invention.
Fig. 2 is a sectional view of the lower leg member of the prosthesis of Fig. 1.
Fig. 3 shows the internal adaptor of the prosthesis of Fig. 1.
Fig. 4 is a top view of the adaptor of Fig. 3.
Fig. 5 shows a removable mould that can be used during the manufacturing process of the leg prosthesis.

### Detailed description of preferred embodiments

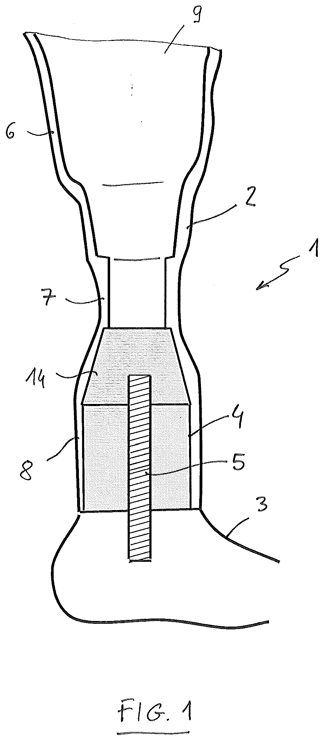

Referring to Fig. 1, a leg prosthesis 1 according to an embodiment of the invention comprises a lower leg member 2, a foot member 3, an internal adaptor 4 and a fixating screw 5.

The leg member 2 is moulded in a single piece and is sufficiently flexible to allow the desired forward movement. Preferably the leg member 2 is made of a suitable polymer and more preferably it is made of polypropylene (PP) or a copolymer of PP and PE.

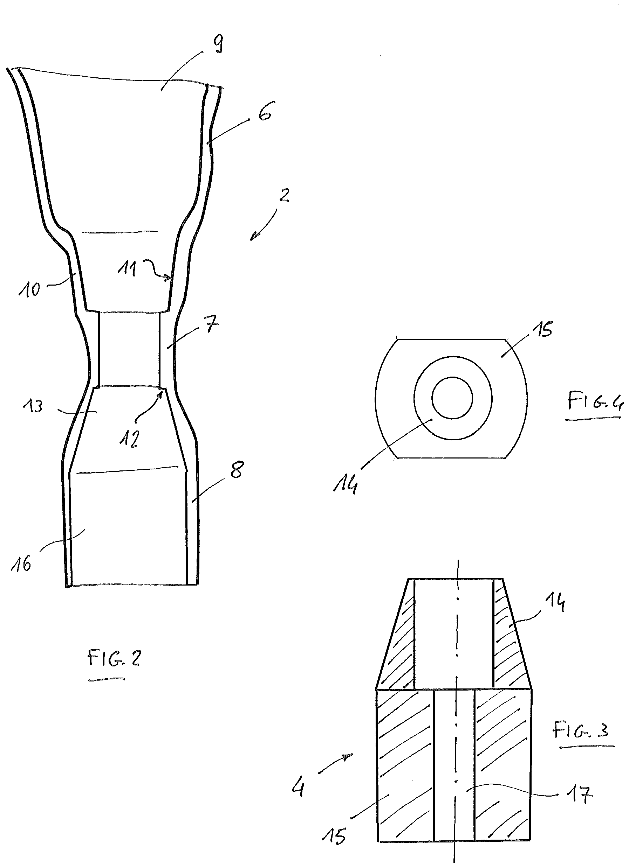

Said leg member 2, which is also illustrated in Fig. 2, comprises an upper portion 6, a middle portion 7, and a lower portion 8.

The upper portion 6 is designed to accommodate the amputation stump of the patient. Said portion 6 is manufactured based on a cast of the patient's leg stump, according to the process that will be described. This portion 6 is custom-made to ensure comfort.

More in detail, said upper portion 6 provides an upper shell with a socket 9 to receive the amputation stump of the patient. The socket 9 is generally of a large diameter but relatively thin, compared to the rest of the leg 2 and especially to the middle portion 7. This portion 6 is designed to distribute forces medially and laterally, and has a wall portion 10 which is advantageously cone-shaped to distribute the load on the middle portion 7 below. In the shown embodiment, said wall portion 10 defines a conical inner surface 11 (Fig. 2).

The middle portion 7 is substantially the load-carrying part of the whole prosthesis 1. To this purpose, said middle portion 7 has a greater thickness than the other parts 6, 8 of the leg member 2. The suitable thickness can be determined according to the weight of the user.

The transition from the thick middle portion 7, to the thinner lower portion 8, as seen in the figures, provides an annular inner surface 12 for abutment of the adaptor 4.

More in detail, the adaptor 4 is fixed to the leg member 2 by means of a conical fitting. The lower portion 8 of leg member 2 comprises a suitable conical seat 13 designed to receive a conical tapered head 14 (Fig. 3) of the adaptor 4, abutting against said annular surface 12.

The seat 13 of the leg member 2 and the head 14 of the adaptor 4, in other words, are the female end and male end of a tight conical fitting, which ensures that the adaptor 4 is firmly fixed into the leg member 2.

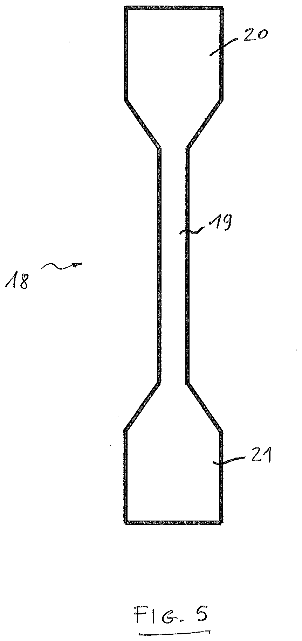

The seat 13 and the conical wall 10 can be obtained during the moulding process by means of an appropriate removable mould, such as the removable mould 18 depicted in Fig. 5.

The adaptor 4 is made of an inelastic material, for example a rigid polyethylene, to support the compression and shear load acting on the leg member 2.

In the most common applications, the adaptor 4 may have a length of around 100 mm and a width or diameter around 50 mm. The cone angle of the tapered head 14, as well as the corresponding cone angle of the seat 13, are preferably in the range 30° to 40°. These measures are given for indicative purpose and are not limiting.

The figures show an embodiment where the adaptor 4, in addition to the tapered head 14, has a base portion 15 which is larger than the tapered head 14, and is received in a lower aperture 16 of the leg member 2, below the conical seat 13. Said base portion 15 has a passing through hole 17 for the fixation screw 5 of the foot member 3.

The foot member 3 is preferably a standardized SACH foot. Indeed, there is no need of a more expensive prosthetic foot, thanks to the good flexibility given by the flexible leg member 2.

The fixation screw 5 is also a standardized component, for example a threaded rod having a diameter of 10 mm and a length of around 100 mm.

A process for the making of the leg 1 is now described in detail.

### Preliminary evaluation

The process starts with a preliminary evaluation, which is done by a chief prosthetist to examine the stump and evaluate whether or not the stump is ready for receiving the leg prosthesis. The joint is also evaluated for flexion or extension contracture to see how well the joint is and will respond accordingly.

In case of a positive result, it is possible to proceed to the next step. The chief prosthetist will normally delegate various tasks to a prosthetist or a practitioner who will be responsible of taking the cast needed for the mould, and select a technician who will be responsible for fabrication of the soft liner (socket), cutting the plastic sheet and taking care of the overall fabrication process.

### Measurements and negative casting

Firstly, a wet stockinette is applied on the stump. Then the practitioner draws a number of landmarks on specific parts of the stump, e.g. with a copy pencil. Preferably, said landmarks include tibial crest, head of fibula, patellar tendon, patella, scars (if any), and protuberances. After this, the practitioner applies anterior and lateral alignment lines and ties a knot in a position where the mandrill (half inch pipe holding the mould) will go out of the cast to the specific length of the whole projected leg.

Then, the practitioner takes a negative stamp of plaster of Paris, making sure that it is applied tightly to precisely copy landmarks on the interior face of the cast. The plaster will dry in minutes (5-7 minutes). The dried cast is removed and is transferred to a plaster room.

### Positive casting

A mandrill of a required length is passed through the hollow negative cast, fixed with a clamp and properly aligned. Then it is put in the upright position, perpendicular to the lateral and anterior lines, and fixed to a vice.

A suitable powder of plaster of Paris is poured into the negative cast. The plaster will cure in about ten minutes. The negative is removed and a positive casting is obtained. The positive casting is then finished by the prosthetist, building up (with some extra plaster) or shaving plaster where necessary. It should be noted that this positive casting reproduces the desired shape, in particular the contour of the upper portion 6 of the leg member 2, to match with the stump of the patient.

### Preparation of the positive casting

The positive casting, as obtained in the previous step, is coated with a soft liner. Said soft liner is preferably a rubber sheet. The material of the soft liner is preferably selected between: EVA, Evalux, TP foam, light PE. Preferably the soft liner is about 5 mm thick. Said soft liner is cut to fit the mould, and then is sanded. The edges of the soft liner are glued to make a cone, then they are heated to about 100 degrees to ease the proper fitting on the mould. The heated liner is moulded on the positive cast, so that it will take the desired shape.

Then, an adaptor system is introduced into the mould, for example the adaptor 18 of Fig. 5. Said adaptor system is a standardized piece which provides the mould for realization of the portions 7, 8 of the leg member 2. Said adaptor system comprises a pipe 19, an upper cone 20 removably connected to the pipe, and a lower cone 21 also removably connected to said pipe. The cones are made for example of nylon. They are joined together by the pipe, and removed after the moulding process is done. It can be appreciated that the cones 20, 21 form the portions 10 and 13 of the leg member 2 (see e.g. Fig. 2).

### Moulding of the leg member

A sheet of the desired plastic material, for example PP, is cut to the desired size and shape, and then heated in a flat-bed oven at a suitable temperature, which is generally in the range 200 to 300 degrees. After about ten to fifteen minutes, the plastic sheet becomes transparent. Transparency indicates that the plastic sheet is ready to be draped.

Draping is the process of putting the ready plastic sheet onto the mold like a covering which will stick together sealing the whole mold. In this stage it is preferred to evacuate air with a vacuum machine, to obtain the exact shape desired.

While the plastic is still warm, the extremities are cut and the piece is let to cool for about twenty minutes. After that, the internal adaptor system 18 is removed and the cast is evacuated, braking and removing the plaster which served as the positive mould.

### Finishing and assembly

The leg member 2 is finished, smoothening and trimming the edges. This can be done on a simple machining facility like a router machine. The foot member 3 is attached to the adaptor 4 with an internal bolt, and mounted into the leg. The prosthesis can be finished e.g. with an EVA coating to give the impression of a natural leg. The prosthesis is now ready and can be fitted to the patient.

The leg prosthesis meets the above stated aims and purposes. It is affordable, accurate and light. The process is easy to implement also when scarce resources are available. The process is also fast, since it may take around 4 hours from the first step of evaluation to the complete prosthesis ready to use.

## Claims

1. A leg prosthesis (1) comprising a lower leg member (2) and a foot member (3), wherein said lower leg member (2) is moulded in a single piece; said leg member (2) is a single-piece hollow body comprising an upper portion (6) suitable to accommodate an amputation stump of a patient; a middle portion (7) which forms a hollow pylon of the leg member; a lower portion (8) for connection with the foot member; said leg prosthesis (1) also comprises an adaptor (4) which is received in a seat (13) of the lower leg member (2), and said foot member (3) is fixed to said adaptor (4), **characterized in that** said lower leg member (2) is made of a flexible material and **in that** said middle portion (7) has the smallest outside dimension but the greatest wall thickness compared to said upper (6) and lower (8) portions of the same leg member (2).

2. A leg prosthesis according to claim 1, wherein said adaptor (4) comprises a male part (14) which is tightly fitted into said seat (13) of the lower leg member.

3. A leg prosthesis according to claim 2, comprising a conical fitting between said male part of the adaptor and said seat of the lower leg member.

4. A leg prosthesis according to claim 3, said adaptor (4) comprising a tapered head (14) having a frusto-conical shape, and said leg member (2) comprising a conical seat (13) dimensioned to match said head of the adaptor with a tight fitting.

5. A leg prosthesis according to any of the preceding claims, said lower leg member (2) being flexible in the direction of the forward movement of a patient using the prosthesis.

6. A leg prosthesis according to any of the previous claims, said leg member (2) being made of polypropylene or of a copolymer of polypropylene and polyethylene.

7. A leg prosthesis according to claim 6, said copolymer of polypropylene and polyethylene containing 10% to 50% polyethylene, and preferably around 20%.

8. A leg prosthesis according to any of the previous claims, said upper portion (6) of the leg member (2) having a cone-shaped wall section (10) at the transition between said upper portion and said middle portion, to uniformly distribute the load during the use.

9. A leg prosthesis according to any of the previous claims, wherein the connection between said adaptor (4) and foot member (3) is made through a fixating screw (5).

10. A leg prosthesis according to any of the previous claims, said adaptor (4) being made of an inelastic material.

11. A leg prosthesis according to any of the previous claims, wherein said foot member (3) is a SACH foot prosthesis.

## Patentansprüche

1. Eine Beinprothese (1), die ein Unterschenkelteil (2) und einen Fußteil (3) aufweist, wobei der Unterschenkelteil (2) in einem Stück ausgeformt ist;
wobei der Unterschenkelteil (2) ein einstückiger, hohler Körper ist, der einen oberen Abschnitt (6), der geeignet ist, einen Amputationsstumpf eines Patienten aufzunehmen; einen mittleren Abschnitt (7), der einen hohlen Pylon des Unterschenkelteils ausbildet; und einen unteren Abschnitt (8) für die Verbindung mit dem Fußteil aufweist; wobei die Beinprothese (1) auch einen Adapter (4) aufweist, der in einem Sitz (13) des Unterschenkelteils (2) aufgenommen ist, wobei der Fußteil (3) an dem Adapter (4) befestigt ist, **dadurch gekennzeichnet,**
**dass** der Unterschenkelteil (2) aus einem flexiblen Material besteht und dass der mittlere Abschnitt (7) die kleinste Außenabmessung aber die größte Wanddicke verglichen mit dem oberen Abschnitt (6) und dem unteren Abschnitt (8) des gleichen Unterschenkelteils (2) hat.

2. Eine Beinprothese nach Anspruch 1, worin der Adapter (4) ein Steckteil (14) hat, das fest in dem Sitz (13) des Unterschenkelteils eingesetzt ist.

3. Eine Beinprothese nach Anspruch 2, die eine konische Muffe zwischen dem Steckteil des Adapters und dem Sitz des Unterschenkelteils aufweist.

4. Eine Beinprothese nach Anspruch 3, wobei der Adapter (4) einen konisch zulaufenden Kopf (14) mit einer kegelstumpfförmigen Form aufweist und wobei der Unterschenkelteil (2) einen konischen Sitz (13) aufweist, der so ausgelegt ist, dass er zu dem Kopf des Adapters mit einer eng anliegenden Muffe passt.

5. Eine Beinprothese nach einem der vorhergehenden Ansprüche, wobei der Unterschenkelteil (2) in der Richtung der Vorwärtsbewegung eines Patienten flexibel ist, der die Prothese verwendet.

6. Eine Beinprothese nach einem der vorhergehenden Ansprüche, wobei der Unterschenkelteil (2) aus Polypropylen or einem Copolymer von Polypropylen und Polyethylen besteht.

7. Eine Beinprothese nach Anspruch 6, wobei der Copolymer aus Polypropylen und Polyethylen 10% bis 50% und bevorzugt ungefähr 20% von Polyethylen enthält.

8. Eine Beinprothese nach einem der vorhergehenden Ansprüche, wobei der obere Abschnitt (6) des Unterschenkelteils (2) einen konusförmigen Wandabschnitt (10) an dem Übergang zwischen dem oberen Abschnitt und dem mittleren Abschnitt hat, um die Last während der Verwendung gleichmäßig zu verteilen.

9. Eine Beinprothese nach einem der der vorhergehenden Ansprüche, worin die Verbindung zwischen dem Adapter (4) und dem Fußteil (3) durch eine Befestigungsschraube (5) bewerkstelligt ist.

10. Eine Beinprothese nach einem der vorhergehenden Ansprüche, wobei der Adapter (4) aus einem unelastischen Material besteht.

11. Eine Beinprothese nach einem der vorhergehenden An-sprüche, worin der Fußteil (3) eine SACH-Fußprothese ist.

## Revendications

1. Prothèse de jambe (1) comprenant un élément de jambe inférieure (2) et un élément de pied (3), où le dit élément de jambe inférieure (2) est moulé en une pièce unique, le dit élément de jambe (2) est un corps creux d'une seule pièce comprenant une partie supérieure (6) adaptée à accueillir le moignon d'amputation d'un patient, une partie médiane (7) qui forme le pilon creux de l'élément de jambe, une partie inférieure (8) destinée à la connexion avec l'élément de pied, la dite prothèse de jambe (1) comprend également un adaptateur (4) qui est reçu dans une assise (13) de l'élément de jambe inférieure (2), et le dit élément de pied (3) est fixé au dit adaptateur (4), **caractérisée en ce que** le dit élément de jambe inférieure (2) est fait dans un matériau flexible et **en ce que** la dite partie médiane (7) présente la dimension extérieure la plus petite mais l'épaisseur de paroi la plus grande par rapport aux dites parties supérieure (6) et inférieure (8) du même élément de jambe (2).

2. Prothèse de jambe selon la revendication 1, **caractérisée en ce que** le dit adaptateur (4) comprend une pièce mâle (14) qui est fermement encastrée dans la dite assise (13) de l'élément de jambe inférieure.

3. Prothèse de jambe selon la revendication 2, comprenant une pièce conique entre la dite pièce mâle de l'adaptateur et la dite assise de l'élément de jambe inférieure.

4. Prothèse de jambe selon la revendication 3, le dit adaptateur (4) comprenant une tête conique (14) ayant une forme tronconique, et le dit élément de jambe (2) comprenant une assise conique (13) dimensionnée afin de correspondre à la dite tête de l'adaptateur avec un encastrement ajusté.

5. Prothèse de jambe selon l'une quelconque des revendications précédentes, le dit élément de jambe inférieure (2) étant flexible dans la direction du mouvement vers l'avant d'un patient utilisant la prothèse.

6. Prothèse de jambe selon l'une quelconque des revendications précédentes, où le dit élément de jambe (2) étant constitué de polypropylène ou d'un copolymère de polypropylène et de polyéthylène.

7. Prothèse de jambe selon la revendication 6, où le dit copolymère de polypropylène et de polyéthylène contient de 10% à 50% de polyéthylène, et de préférence environ 20%.

8. Prothèse de jambe selon l'une quelconque des revendications précédentes, où la dite partie supérieure (6) de l'élément de jambe (2) présente une section de paroi en forme de cône (10) au niveau de la transition entre la dite partie supérieure et la dite partie médiane, afin de distribuer uniformément la charge pendant l'utilisation.

9. Prothèse de jambe selon l'une quelconque des revendications précédentes, **caractérisée en ce que** la connexion entre le dit adaptateur (4) et l'élément de pied (3) est réalisée par une vis de fixation (5).

10. Prothèse de jambe selon l'une quelconque des revendications précédentes, le dit adaptateur (4) étant fait d'un matériau non élastique.

11. Prothèse de jambe selon l'une quelconque des revendications précédentes, **caractérisée en ce que** le dit élément de pied (3) est une prothèse de pied SACH.
